# EUROPEAN PATENT SPECIFICATION

(11) **EP 4 117 686 B1**
(45) Date of publication and mention of the grant of the patent: **04.12.2024**
(21) Application number: 21766967.0
(22) Date of filing: 10.03.2021
(51) Int. Cl.: A61K 9/127, A61K 33/24

(54) **LIPOSOME COMPOSITION AND PREPARATION METHOD THEREOF**
LIPOSOMZUSAMMENSETZUNG UND HERSTELLUNGSVERFAHREN DAFÜR
COMPOSITION LIPOSOMALE ET SON PROCÉDÉ DE PRÉPARATION

(30) Priority: 10.03.2020 US 202062987366 P
(43) Date of publication of application: 18.01.2023
(73) Proprietor: Chung Yuan Christian University, Taoyuan City, 320 (TW)
(72) Inventor: HSU, Yih-Chih, Taoyuan City, 320 (TW); GUSTI NGURAH PUTU, Eka, Putra, Taoyuan City, 320 (TW)
(74) Representative: Viering, Jentschura & Partner mbB Patent- und Rechtsanwälte
(86) International application number: PCT/US2021/021605
(87) International publication number: WO 2021/183588

(56) References cited:
- WO-A2-2006/084248
- US-A1- 2004 101 553
- US-A1- 2007 160 656
- US-A1- 2013 011 466
- US-A1- 2013 011 466
- NEWMAN M.S. ET AL.: "COMPARATIVE PHARMACOKINETICS, TISSUE DISTRIBUTION AND THERAPEUTIC EFFECTIVENESS OF CISPLATIN ENCAPSULATED IN LONG-CIRCULATING, PEGYLATED LIPOSOMES (SPI-077) IN TUMOR-BEARING MICE", CANCER CHEMOTHERAPY AND PHARMACOLOGY, SPRINGER VERLAG , BERLIN, DE, vol. 43, 1 January 1999 (1999-01-01), pages 1 - 07, XP001018172, ISSN: 0344-5704, DOI: 10.1007/S002800050855
- DASARI ET AL.: "Cisplatin in cancer therapy: molecular mechanisms of action", EUR J PHARMACOL., vol. 740, 5 October 2014 (2014-10-05), pages 364 - 378, XP055474371, DOI: 10.1016/j.ejphar. 2014.07.02 5

## Description

### BACKGROUND

### Technical Field

The present disclosure relates to a method for preparing a liposome composition encapsulating a platinum-based drug, as set out in the claims.

### Related Art

cis-diaminedichloroplatinum (II) (CDDP, also known as cisplatin) has been commonly used as a chemotherapeutic agent. However, it is poorly water soluble, and its high toxicity is known to cause various undesirable side effects.

### SUMMARY

To improve solubility and reduce toxicity of CDDP, present disclosure provides a method for preparing a liposome composition. The method comprises the steps of: providing precursor liposomes encapsulating platinum-based precursors; and incubating the precursor liposomes with a salt solution to convert the platinum-based precursors to platinum-based drugs to form the liposome composition.

Drug loading of the liposome composition is at least 10%.

According to the present disclosure, the liposome composition provides an effective solution to improving platinum-based drug solubility and encapsulation efficiency by liposomal particles. By using the methods of the embodiments of the present disclosure, the precursor liposomes can be converted to platinum-based drug encapsulating liposomes with ease and at low cost. The methods also provide an effective tool for enhancing drug loading of liposome compositions.

### BRIEF DESCRIPTION OF THE DRAWINGS

The disclosure will become more fully understood from the detailed description given herein below for illustration only, and thus not limitative of the disclosure.
FIG. 1 illustrates a schematic diagram of a preparation of a liposome composition according to an exemplary embodiment of the present disclosure;
FIG. 2 illustrates a preparation of a CDDP precursor of the liposome composition according to an exemplary embodiment of the present disclosure;
FIG. 3 illustrates a schematic diagram of a preparation of a liposome composition according to an exemplary embodiment of the present disclosure;

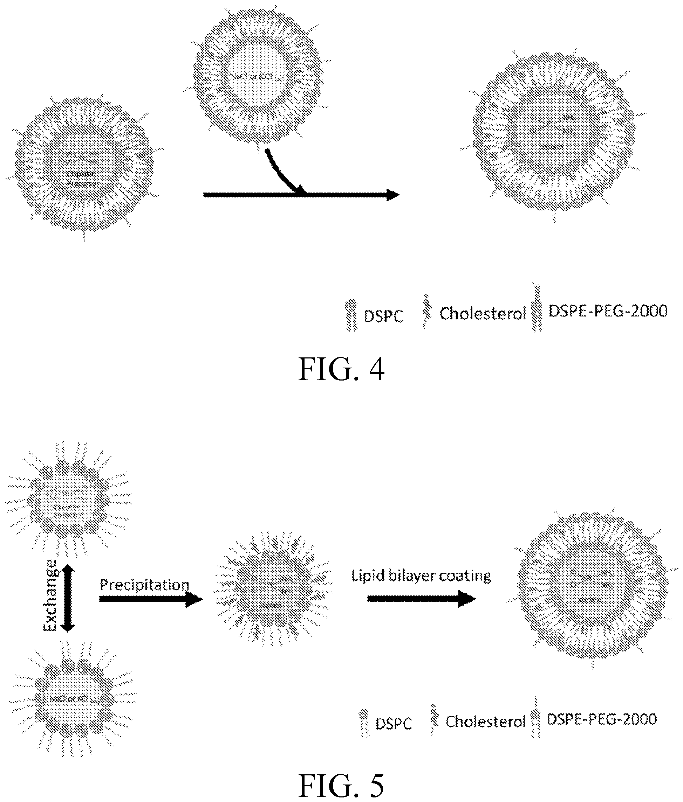

FIG. 4 illustrates a schematic diagram of a preparation of the liposome composition according to an exemplary embodiment of the present disclosure;
FIG. 5 illustrates a schematic diagram of a preparation of the liposome composition according to an exemplary embodiment of the present disclosure;
FIG. 6 shows a Cryo-EM image of the liposome composition according to an exemplary embodiment of the present disclosure;
FIG. 7 shows a size distribution of the liposome composition according to an exemplary embodiment of the present disclosure;
FIG. 8 shows a pharmacokinetic analysis of the liposome composition in animal models according to an exemplary embodiment of the present disclosure;
FIG. 9 shows changes in tumor growth and tumor volume of human non-small-cell-lung-cancer (NSCLC) adenocarcinoma H1975 cells xenograft mouse models treated with PBS, CDDP, and LipoCis;
FIG. 10 shows changes in tumor growth, tumor volume and body weight of A549 xenograft mouse models treated with PBS and LipoCis;
FIG. 11 shows dose-dependent changes in tumor volume, body weight, tumor growth and weight change of lung large cell adenocarcinoma H460 cells xenograft mouse models treated with PBS and LipoCis;
FIG. 12 shows changes in tumor volume of human oral squamous cell carcinoma (HOSCC) SAS cells xenograft mouse models treated with PBS, CDDP, and LipoCis; and
FIG. 13 shows the tumor reduction effect of the liposome composition according to an exemplary embodiment of the present disclosure in the metastatic SAS cells of HOSCC mouse models.

### DETAILED DESCRIPTION

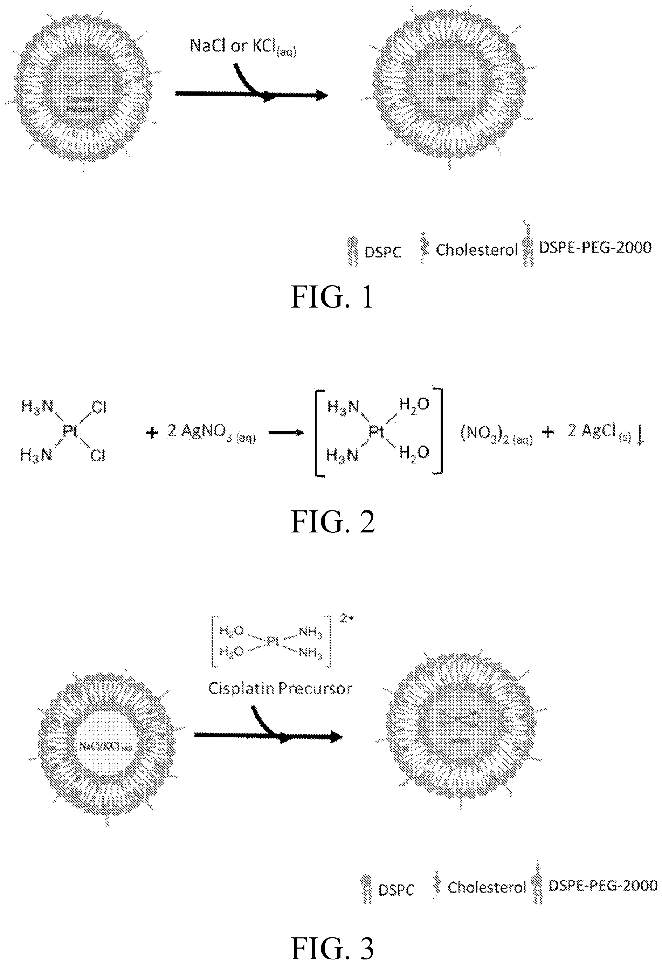

Referring to FIG. 1. A method for preparing a liposome composition is provided. The method comprises the steps of: providing precursor liposomes encapsulating platinum-based precursors; and incubating the precursor liposomes in a salt solution to convert the platinum-based precursors to platinum-based drugs to form the liposome composition. Specifically, the precursor liposomes are prepared by the steps of: hydrating the platinum-based drugs to form the platinum-based precursors; and adding the platinum-based precursors to a lipid bilayer vehicle to form the precursor liposomes.

The platinum-based drugs are hydrated by incubating the platinum-based drugs with silver nitrate (AgNO₃), silver sulfate (Ag₂SO₄), silver phosphate (Ag₃PO₄), calcium nitrate (Ca(NO₃)₂), calcium sulfate (CaSO₄), calcium phosphate (Ca₃(PO₄)₂), magnesium nitrate (Mg(NO₃)₂), magnesium sulfate (MgSO₄), and/or magnesium phosphate (Mg(H₂PO₄)₂).

The platinum-based drugs comprise at least one platinum-halides bond (e.g., Pt-F, Pt-Cl, Pt-Br, or Pt-I bonds). Some examples of the platinum-based drugs may include cisplatin, triplatin, phenanthriplatin, picoplatin, satraplatin, *cis*-diamine diiodo platinum (II), *cis*-diamine difluoro platinum (II), and *cis*-diamine dibromo platinum (II).

Referring to FIG. 2. The platinum-based precursors may be monoaqua and/or diaqua forms of the platinum-based drugs; for example, *cis*-[Pt(NH₃)₂(H₂O)₂](NO₃)₂ or *cis-*[Pt(NH₃)₂(H₂O)₂]²⁺. The platinum-based precursors may be encapsulated into the lipid bilayer vehicle (e.g., liposomal nanoparticles) due to their water-soluble nature.

The lipid bilayer vehicle may be prepared by mixing a lipid-based formulation in an organic solvent, such as chloroform, cyclohexane, methanol, ethanol, ethyl acetate, or any combination thereof. The lipid-based formulation may include a combination of choline phospholipids, cholesterols, and polyethylene glycol (PEG)-based compounds. Preferably, the choline phospholipids may include neutral lipids, such as 1,2-distearoyl-sn-glycero-3-phosphocholine (DSPC), 1,2-dioleoyl-sn-glycero-3-phosphocholine (DOPC), 1,2-dipalmitoyl-sn-glycero-3-phosphocholine (DPPC), 1,2-dilauroyl-sn-glycero-3-phosphocholine (DLPC), 1,2-dimyristoyl-sn-glycero-3-phosphocholine (DMPC), hexadecyl phosphorylcholine (HePC), 1-stearoyl-2-oleoyl-sn-glycero-3-phosphocholine (SOPC), 1,2-diphytanoyl-sn-glycero-3-phosphocholine (diPhyPC), or any combination thereof. The PEG-based compounds may be distearoylphosphatidyl ethanolamine (DSPE)-PEG compounds, such as [N-(carbonyl-methoxypolyethylene glycol-200)-1,2-distearoyl-sn-glycero-3-phosphoethanolamine (DSPE-PEG-200 or DSPE-mPEG-200), DSPE-PEG-400 (or DSPE-mPEG-400), DSPE-PEG-800 (or DSPE-mPEG-800), DSPE-PEG-1000 (or DSPE-mPEG-1000), DSPE-PEG-2000 (or DSPE-mPEG-2000), DSPE-PEG-2500 (or DSPE-mPEG-2500), DSPE-PEG-3000 (or DSPE-mPEG-3000), DSPE-PEG-4000 (or DSPE-mPEG-4000), DSPE-PEG-5000 (or DSPE-mPEG-5000), DSPE-PEG-6000 (or DSPE-mPEG-6000), DSPE-PEG-10000 (or DSPE-mPEG-10000), or any combination thereof. In some embodiments, the PEG-based compounds may be selected from DSPE-PEG-aminoethyl anisamide (DSPE-PEG-AEAA), DSPE-PEG-monoclonal antibodies (DSPE-PEG-mAb), and DSPE-PEG with other ligand moieties.

The lipid-based formulation may be self-assembled in an aqueous environment via hydrophobic interaction and/or van der Waals interaction to form the lipid bilayer vehicle. In one or more preferred embodiments, neutrality of the lipid-based formulation provides minimum energy bonding to the encapsulated active pharmaceutical ingredients (API) or precursors thereof, therefore facilitating drug release *in vivo.* Furthermore, as the neutral lipid bilayer vehicle does not interact with the charged precursors, drug conversion occurred therein would not be affected or hindered.

In the embodiment, the volume ratio of the lipid bilayer vehicle to the CDDP precursors in the liposome composition may fall within 1:1 to 20:1. The molar ratio of the CDDP precursors to the lipid bilayer vehicle may fall within 0.1:1 to 1:1. The CDDP precursors may be added to the lipid bilayer vehicle at an oil to water ratio of 1:0.01 to 1:0.8. The concentration of the platinum-based precursors added to the lipid bilayer vehicle or in the resulting precursor liposomes may fall within a range of 25 mM to 600 mM, preferably 1.5 mM to 5 mM.

To convert the platinum-based drugs from the platinum-based precursors, the precursor liposomes are incubated in the salt solution to allow the salts to enter the precursor liposomes. In the embodiment, the salt solution may include fluoride, chloride, bromide, iodide, or other salts of the halogen group. A concentration of the salt solution may fall within a range of 0.2 M to 4 M; more specifically, when NaCl is used for the conversion, the concentration of NaCl may fall within a range of 0.4 M to 3.9 M; when KCl is used, the concentration of KCl may fall within a range of 0.4 M to 3.0 M. In an embodiment, the incubation may be carried out at 4-65°C for 1-24 h to allow the salts to enter the precursor liposomes and convert CDDPs from diaqua CDDP precursors. For example, the precursor liposomes may be incubated with the salt solution at 4-7°C overnight, or at 10-50°C for 2-15 h followed by cooling to stabilize the structure of the liposome composition.

In one or more embodiments, the high concentration of the salt solution generates an osmotic pressure that pushes the halogen ions through the lipid bilayer vehicle irreversibly and allows the halogen ions to stay inside of the precursor liposomes, without affecting the stability of the liposome structure. As the halogen ions are being consumed inside the precursor liposomes for API conversion, more halogen ions would continue to diffuse into the precursor liposomes. Such osmosis-based approach presents a cost-effective and time-efficient route for driving drug conversion inside the precursor liposomes.

In an example, to prepare a liposome composition (abbreviated hereunder as LipoCis) in which CDDP is the platinum-based drug encapsulated in a lipid bilayer vehicle made of DSPC, cholesterol, and DSPE-PEG-2000, CDDP precursors may be obtained by incubating 0.2-0.4 mmol of CDDP with 0.3-0.4 mmol of silver nitrate (AgNO_{3(aq)}) at 25°C for 16-18 h or at 60°C for 3-4 h. Thereafter, the LipoCis may be formed firstly by mixing DSPC, cholesterol, and 40-50:25-50:10-30 w/w% of DSPE-PEG-2000 at 30-60°C under 100-400 rpm for 10-60 min to form the lipid bilayer vehicle. The CDDP precursors may then be added into the lipid bilayer vehicle at the v/v ratio of 1:0.01 to 1:0.8 oil-to-water by using a micro-volume dropper at 1 mL/min or by bulk mixing followed by either handshaking or stirring for 15-30 min to form the precursor liposomes. The liposome encapsulating the CDDP precursors was then homogenized for 1-10 passes to reach a liposome size of 60-250 nm. Finally, the CDDP precursors in the liposomes were converted to CDDPs by incubating the precursor liposomes in 0.2-3.9M of potassium chloride (KCl) or sodium chloride (NaCl) at 25-50°C and stirring for about 2-24 h. The resulting LipoCis may be purified by using a tangential flow filtration (TFF) system to remove excess salts and be exchanged into a 10 mM HEPES, 5% glucose buffer (pH 6.5-7.6), a 10 mM HEPES, 0.9% saline buffer (pH 6.5-7.6), a 0.9% saline solution, 5% glucose solution, or ddH₂O for storage. The drug-to-lipid (D/L) ratio of the resulting LipoCis may range up to 0.05-0.8 per mole.

As evidenced by the high conversion rate and drug loading shown in Table 1, the method of the present disclosure as mentioned above can effectively encapsulate and convert the platinum-based precursors to the platinum-based drugs. Accordingly, the calculated drug loading of the liposome composition prepared by the embodiments of the present disclosure could be as high as 62%.

**Table 1. Drug loading (DL) of the LipoCis of the embodiments**

| Sample | CDDP Con. (mg/mL) from HPLC | CDDP Con. (mg/ml) from ICP | Conversion (%) | DL (%) |
|---|---|---|---|---|
| LipoCis with NaCl | 0.38 | 0.41 | 93 | 62 |
| LipoCis with KCl | 0.36 | 0.40 | 90 | 62 |

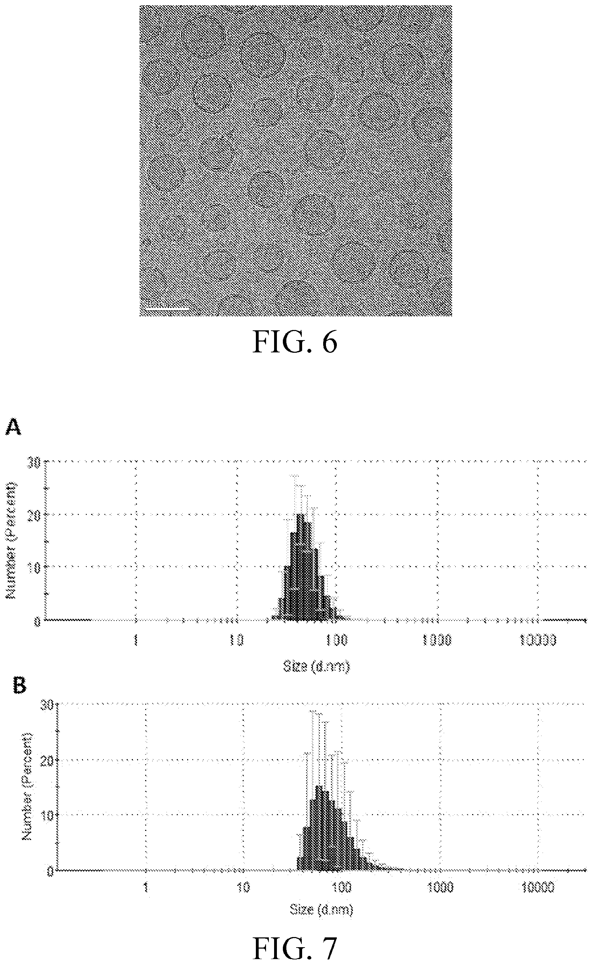

Referring to FIGS. 6 and 7. In an example, CDDP is encapsulated and precipitated into a lipid bilayer made of DSPC, cholesterol and DSPC-PEG-2000 to form LipoCis nanoparticles (NPs). The LipoCis NPs may be characterized by various approaches. In the example shown in FIGS. 6 and 7, particle size and zeta potential were measured using the Malvern Zetasizer Nano series (Westborough, MA, US). Morphology of the LipoCis NPs was captured using cryogenic electron microscopy (Cryo-EM). The amount of CDDP was measured using high performance liquid chromatography (HPLC) and the Pt content in the LipoCis NPs was validated using inductively coupled plasma-atomic emission spectroscopy (ICP-AES) or inductively coupled plasma-optical emission spectrometry (ICP-OES). The excipient concentration was determined using HPLC evaporative light scattering detectors (ELSD). As shown in FIG. 6, the morphology of the LipoCis NPs as captured by Cryo-EM revealed a fine and homogeneous monodispersity, with an approximate particle size in the range 80-150 nm, which is equivalent to that of dynamic light scattering (DLS) measurement. As shown in FIG. 7, all interaction polymer chromatography (IPC) points of the LipoCis NPs were measured.

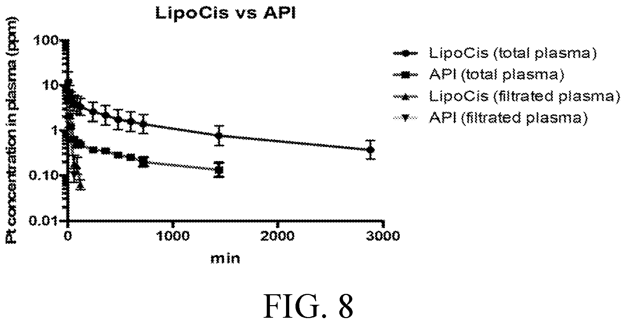

Referring to FIG. 8. A pharmacokinetic analysis of the LipoCis prepared according to the first embodiment of the present disclosure was performed in a rat model.

As shown in Table 2, the LipoCis resulted in low *in vivo* clearance (CL), high area under the curve (AUC), and long circulatory time (i.e., Vz and Vss values were lower than those of API). No statistically significant difference was observed between LipoCis and API in their half-life and mean residence time (MRT). The pharmacokinetic results suggested a sustained release of the LipoCis *in vivo.*

**Table 2**

| | CL (mL/min) | Vz (mL) | Vss (mL) | AUC (µg-min/mL) | half-life (min) | MRT (min) |
|---|---|---|---|---|---|---|
| LipoCis (n=3) | 0.31±0.26 | 566±570 | 482±485 | 4047±2381 | 1164±245 | 1434±335 |
| API (n=3) | 1.39±0.28 | 1463±250 | 1278±359 | 623±140 | 771±312 | 987±511 |

To assess the inhibitory potential of LipoCis on tumor growth, xenograft experiments were conducted for 21 days and the xenograft animal models were monitored daily. In the experiments, human cancer cell lines (1×10⁶ cells/in 200 µL PBS-Martigel 1:1 solution) were subcutaneously injected in the right hind legs of Balb/c nude mice. After a considerably sized tumor had appeared, the tumor size is measured daily or every other day and calculated by the formula (length×width×height)/2. When the tumor size reached to the desired size (e.g., 100-210 mm³), LipoCis samples were intravenously injected into the tumor-bearing mice once per week for 3 weeks.

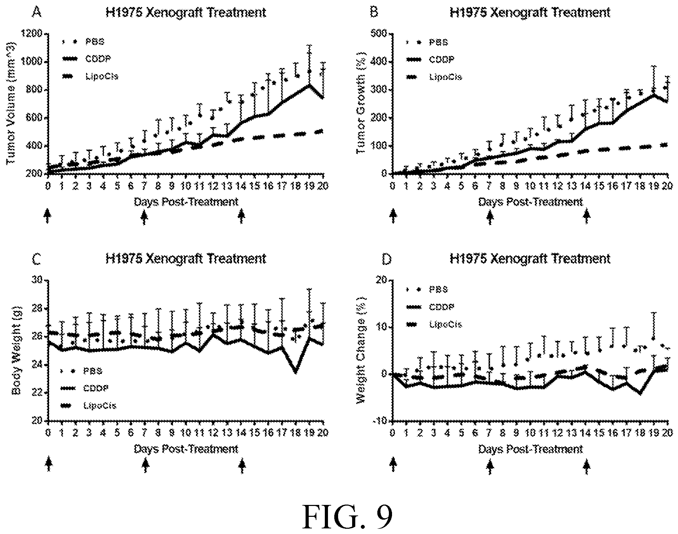

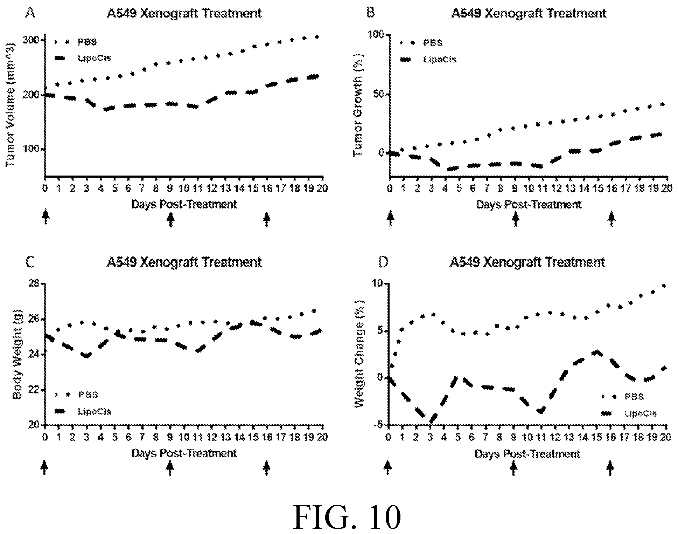

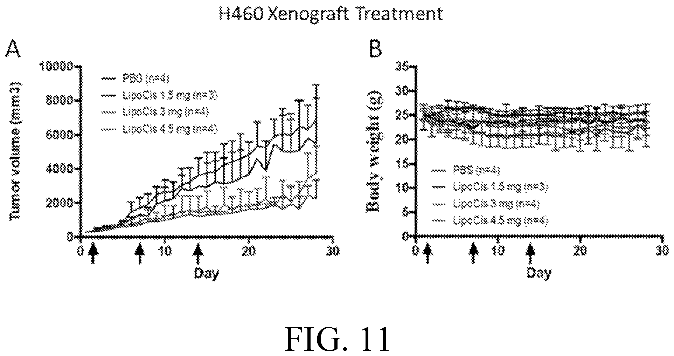

Referring to FIGS. 9 to 11. Three lung cancer cell lines, including human non-small-cell-lung-cancer adenocarcinoma (NSCLC) H1975 cells and A549 cells, and human large cell carcinoma H460 cells, were tested to validate the *in vivo* efficacy of the LipoCis NPs. As shown in FIG. 9, in the H1975 lung adenocarcinoma cell xenograft mice models, LipoCis prepared according to the embodiments of the present disclosure was shown to induce a more significant apoptotic response (by immunostaining studies) as well as stronger tumor inhibitory effects than CDDP. As shown in FIGS. 10 to 11, similar results were also observed in the A549 lung adenocarcinoma cells and H460 lung large cell carcinoma cells xenograft mice models.

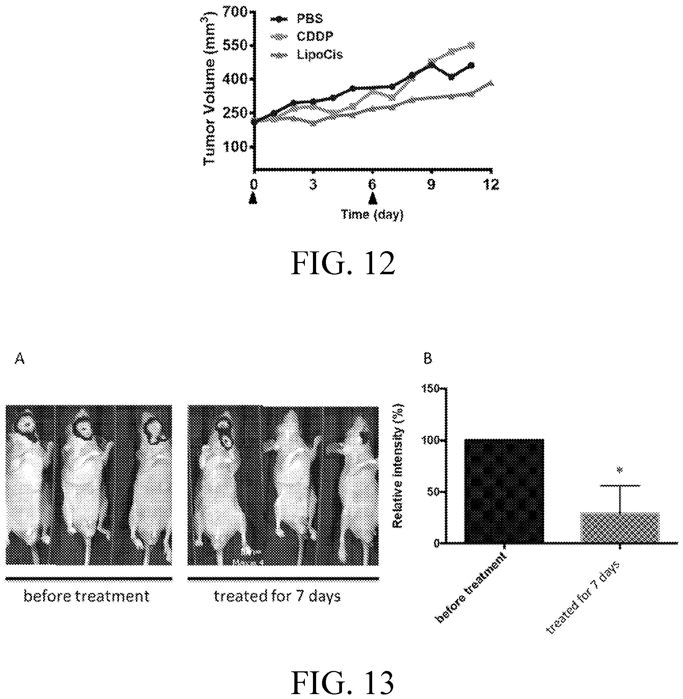

Referring to FIGS. 12 and 13. A human oral squamous cell carcinoma (HOSCC) xenograft animal model was also established. 160 µL of 5 × 10⁶ SAS human oral cancer cells in the presence of 200 µL matrigel (Corning, Bedford, MA) were subcutaneously injected using a 28-gauge needle at the lower right dorsal flank of 7 to 9-week-old male BALB/cAnN.Cg-Foxn1nu nude mouse (from National Laboratory Animal Center, Taipei, Taiwan). The SAS xenograft mice were randomly separated into three groups and treated with (i) phosphate-buffered saline (PBS); (ii) CDDP; and (iii) LipoCis NPs. All of the treatments were administered intravenously. The CDDP or LipoCis NPs were given at a similar dose of 3.0 mg/kg. The treatment procedure was carried out when tumors reached 200.1 mm³ ± 3.5 (or 195-210 mm³). Tumor volume was determined as length × width × high × 0.5. The mice were sacrificed on the 12th day. Excised tumors and organs were dissected and fixed in 10% formalin for further experiments. These studies were approved and carried out in strict accordance with the recommendations in the Guide for the Care and Use of the Institutional Animal Care and Use Committee of Chung Yuan Christian University, Chungli, Taoyuan, Taiwan, ROC.

To examine the efficacy of the LipoCis NPs *in vivo*, the SAS human oral tumor-bearing xenograft models with 200.1±3.5mm³ tumor volume were randomly clustered into three different treatment groups, including (i) PBS; (ii) CDDP; and (iii) LipoCis. Each group received two cycles of treatment with a 6-day interval between each cycle. As demonstrated by the results shown in FIGS. 9 and 10, LipoCis served a potential implication in growth inhibition of SAS tumors.

According to the present disclosure as mentioned above, the LipoCis provided an effective solution to improving platinum-based drug solubility and encapsulation efficiency by liposomal particles. By using the method of the present disclosure, the precursor liposomes can be converted to platinum-based drug encapsulating liposomes with ease and at low cost. The methods also provide an effective tool for enhancing drug loading of liposome compositions.

## Claims

1. A method for preparing a liposome composition, comprising steps of:
providing precursor liposomes encapsulating platinum-based precursors; and
incubating the precursor liposomes in a salt solution to convert the platinum-based precursors to platinum-based drugs to form the liposome composition,
wherein the precursor liposomes are prepared by hydrating the platinum-based drugs to form the platinum-based precursors; mixing a lipid-based formulation to form a lipid bilayer vehicle; and adding the platinum-based precursors to the lipid bilayer vehicle to form the precursor liposomes,
wherein the platinum-based drugs comprise at least one platinum-halide bond; and
the lipid-based formulation comprises DSPC, cholesterol, and a member selected from a group consisting of DSPE-PEG-2000, DSPE-PEG-3000, DSPE-PEG-4000, DSPE-PEG-5000, and DSPE-PEG-10000.

2. The method according to claim 1, wherein the platinum-based drugs are hydrated by incubating the platinum-based drugs with at least one compound selected from a group consisting of AgNO₃, Ag₂SO₄, Ag₃PO₄, Ca(NO₃)₂, CaSO₄, Ca₃(PO₄)₂, Mg(NO₃)₂, MgSO₄, and Mg(H₂PO₄)₂.

3. The method according to claim 1, wherein the lipid-based formulation is mixed in an organic solvent comprising chloroform, ethanol, or ethyl acetate.

4. The method according to claim 1, wherein a volume ratio of the lipid bilayer vehicle to the platinum-based precursors falls within a range of 1:1 to 20:1.

5. The method according to claim 1, wherein a molar ratio of the platinum-based drugs to the lipid bilayer vehicle falls within a range of 0.1: 1 to 1:1.

6. The method according to claim 1, wherein the platinum-based precursors are added to the lipid bilayer vehicle at an oil to water ratio falling within a range of 1:0.01 to 1:0.8.

7. The method according to claim 1, wherein the platinum-based drugs are selected from a group consisting of cisplatin, triplatin, phenanthriplatin, picoplatin, and satraplatin.

8. The method according to claim 1, wherein the platinum-based precursors are at least one of monoaqua and diaqua forms of the platinum-based drugs.

9. The method according to claim 1, wherein the salt solution comprises chlorides or bromides.

10. The method according to claim 1, wherein a concentration of the platinum-based precursors in the precursor liposomes falls within a range of 25 mM to 600 mM.

11. The method according to claim 1, wherein a concentration of the salt solution incubated with the precursor liposomes falls within a range of 0.2 M to 4 M.

12. The method according to claim 1, wherein the precursor liposomes are incubated with the salt solution at 4-65°C for 1-24 h.

## Patentansprüche

1. Verfahren zum Herstellen einer Liposomzusammensetzung, welche Schritte aufweist von:
Bereitstellen von Precursorliposomen, welche Platin-basierte Precursor einkapseln; und
Inkubieren der Precursorliposomen in einer Salzlösung, um die Platin-basierten Precursor in Platin-basierte Arzneimittel umzuwandeln, um die Liposomzusammensetzung zu bilden,
wobei die Precursorliposomen hergestellt werden mittels Hydratisieren der Platin-basierten Arzneimittel, um die Platin-basierten Precursor zu bilden; Mischen einer Lipid-basierten Formulierung, um ein Lipid-Doppelschicht-Vehicle zu bilden; und Zugeben der Platin-basierten Precursor zu dem Lipid-Doppelschicht-Vehikel, um die Precursorliposomen zu bilden,
wobei die Platin-basierten Arzneimittel mindestens eine Platin-Halid-Bindung aufweisen; und
die Lipid-basierte Formulierung DSPC, Cholesterin und ein Mitglied, welches ausgewählt ist aus einer Gruppe bestehend aus DSPE-PEG-2000, DSPE-PEG-3000, DSPE-PEG-4000, DSPE-PEG-5000 und DSPE-PEG-10000, aufweist.

2. Verfahren nach Anspruch 1, wobei die Platin-basierten Arzneimittel hydratisiert werden mittels Inkubieren der Platin-basierten Arzneimittel mit mindestens einer Verbindung, welche ausgewählt ist aus der Gruppe bestehend aus AgNO₃, Ag₂SO₄, Ag₃PO₄, Ca(NO₃)₂, CaSO₄, Ca₃(PO₄)₂, Mg(NO₃)₂, MgSO₄ und Mg(H₂PO₄)₂.

3. Verfahren nach Anspruch 1, wobei die Lipid-basierte Formulierung in einem organischen Lösungsmittel gemischt wird, welches Chloroform, Ethanol oder Ethylacetat aufweist.

4. Verfahren nach Anspruch 1, wobei ein Volumenverhältnis des Lipid-Doppelschicht-Vehikels zu den Platin-basierten Precursorn in einen Bereich von 1:1 bis 20:1 fällt.

5. Verfahren nach Anspruch 1, wobei ein molares Verhältnis der Platin-basierten Arzneimittel zu dem Lipid-Doppelschicht-Vehikel in einen Bereich von 0,1:1 bis 1:1 fällt.

6. Verfahren nach Anspruch 1, wobei die Platin-basierten Precursor dem Lipid-Doppelschicht-Vehikel zugesetzt werden bei einem Öl-zu-Wasser-Verhältnis, welches in einen Bereich von 1:0,01 bis 1:0,8 fällt.

7. Verfahren nach Anspruch 1, wobei die Platin-basierten Arzneimittel aus einer Gruppe ausgewählt sind, welche aus Cisplatin, Triplatin, Phenanthriplatin, Picoplatin und Satraplatin besteht.

8. Verfahren nach Anspruch 1, wobei die Platin-basierte Precursor mindestens eine von einer Monoaqua- und einer Diaqua-Form der Platin-basierten Arzneimittel sind.

9. Verfahren nach Anspruch 1, wobei die Salzlösung Chloride oder Bromide aufweist.

10. Verfahren nach Anspruch 1, wobei eine Konzentration des Platin-basierten Precusors in den Precursorliposomen in einen Bereich von 25 mM bis 600 mM fällt.

11. Verfahren nach Anspruch 1, wobei eine Konzentration der Salzlösung, mit welcher die Precusorliposomen inkubiert werden, in einen Bereich von 0,2 M bis 4 M fällt.

12. Verfahren nach Anspruch 1, wobei die Precursorliposomen mit der Salzlösung bei 4-65°C für 1 bis 24 Stunden inkubiert werden.

## Revendications

1. Procédé de préparation d'une composition de liposomes, comprenant les étapes suivantes :
la fourniture de liposomes précurseurs encapsulant des précurseurs à base de platine ; et
l'incubation des liposomes précurseurs dans une solution saline pour convertir les précurseurs à base de platine en médicaments à base de platine afin de former la composition de liposomes,
dans lequel les liposomes précurseurs sont préparés par l'hydratation des médicaments à base de platine pour former les précurseurs à base de platine ; le mélange d'une formulation à base de lipides pour former un véhicule bicouche lipidique ; et l'ajout des précurseurs à base de platine au véhicule bicouche lipidique pour former les liposomes précurseurs,
dans lequel les médicaments à base de platine comprennent au moins une liaison platine-halide ; et
la formulation à base de lipides comprend de la DSPC, du cholestérol et un membre choisi dans un groupe constitué de DSPE-PEG-2000, DSPE-PEG-3000, DSPE-PEG-4000, DSPE-PEG-5000 et DSPE-PEG-10000.

2. Procédé selon la revendication 1, dans lequel les médicaments à base de platine sont hydratés par l'incubation des médicaments à base de platine avec au moins un composé choisi dans un groupe constitué de AgNO₃, Ag₂SO₄, Ag₃PO₄, Ca(NO₃)₂, CaSO₄, Ca₃(PO₄)₂, Mg(NO₃)₂, MgSO₄ et Mg(H₂PO₄)₂.

3. Procédé selon la revendication 1, dans lequel la formulation à base de lipides est mélangée dans un solvant organique comprenant le chloroforme, l'éthanol, ou l'acétate d'éthyle.

4. Procédé selon la revendication 1, dans lequel un rapport volumique du véhicule bicouche lipidique aux précurseurs à base de platine est compris dans une plage de 1:1 à 20:1.

5. Procédé selon la revendication 1, dans lequel un rapport molaire des médicaments à base de platine au véhicule bicouche lipidique est dans une plage de 0,1:1 à 1:1.

6. Procédé selon la revendication 1, dans lequel les précurseurs à base de platine sont ajoutés au véhicule bicouche lipidique à un rapport huile/eau compris dans une plage de 1:0,01 à 1:0,8.

7. Procédé selon la revendication 1, dans lequel les médicaments à base de platine sont choisis dans un groupe constitué par le cisplatine, le triplatine, le phénanthriplatine, le picoplatine et le satraplatine.

8. Procédé selon la revendication 1, dans lequel les précurseurs à base de platine sont au moins l'une parmi des formes monoaqua et diaqua des médicaments à base de platine.

9. Procédé selon la revendication 1, dans lequel la solution saline comprend des chlorures ou des bromures.

10. Procédé selon la revendication 1, dans lequel une concentration des précurseurs à base de platine dans les liposomes précurseurs est comprise dans une plage de 25 mM à 600 mM.

11. Procédé selon la revendication 1, dans lequel une concentration de la solution saline incubée avec les liposomes précurseurs est comprise dans une plage de 0,2 M à 4 M.

12. Procédé selon la revendication 1, dans lequel les liposomes précurseurs sont incubés avec la solution saline à 4-65 °C pendant 1-24 h.
